# EUROPÄISCHE PATENTSCHRIFT

(11) **EP 3 435 899 B1**
(45) Veröffentlichungstag und Bekanntmachung des Hinweises auf die Patenterteilung: **30.09.2020**
(21) Anmeldenummer: 17725499.2
(22) Anmeldetag: 27.03.2017
(51) Int. Cl.: A61B 17/72, A61F 2/36

(54) **IMPLANTIERBARE AUSGLEICHSMANSCHETTE FÜR EINE ENDOPROTHESE**
IMPLANTABLE COMPENSATING SLEEVE FOR AN ENDOPROSTHESIS
MANCHON ADAPTATEUR IMPLANTABLE POUR ENDOPROTHÈSE

(30) Priorität: 29.03.2016 DE 102016003838
(43) Veröffentlichungstag der Anmeldung: 06.02.2019
(73) Patentinhaber: Merete Holding GmbH, 10719 Berlin (DE)
(72) Erfinder: ANAPLIOTIS, Emmanuel, 14195 Berlin (DE)
(74) Vertreter: Bittner, Thomas L.
(86) Internationale Anmeldenummer: PCT/DE2017/000083
(87) Internationale Veröffentlichungsnummer: WO 2017/167323

(56) Entgegenhaltungen:
- DE-A1- 1 805 665
- US-A1- 2004 230 193
- US-A1- 2008 255 560
- US-A1- 2011 306 975

## Beschreibung

Die Erfindung betrifft eine implantierbare Ausgleichsmanschette für eine Endoprothese, insbesondere für eine liegende Endoprothese, zur intramedullären Versorgung einer periprothetischen oder interprothetischen Fraktur.

Aufgrund der hohen Anzahl implantierter Hüft- und Knieprothesen bei steigendem Alter der operierten Patienten nimmt die Häufigkeit periprothetischer oder interprothetischer Frakturen zu.

Periprothetische Frakturen sind Brüche in der Umgebung einer Prothese und treten oftmals in Folge eines Sturzes auf. Auch kann eine schlechte Knochenqualität aufgrund von osteoporotischen oder osteolytischen Veränderungen zu einer periprothetischen Fraktur führen.

Interprothetische Frakturen treten zwischen zwei im selben Knochen einliegenden Implantaten bzw. Prothesen auf. Am häufigsten findet man interprothetische Frakturen im Femur zwischen einer Hüft- und einer Knieprothese.

In Abhängigkeit von Art und Verlauf der Fraktur, deren Ort in Bezug auf die Prothese sowie in Abhängigkeit von der Sitzstabilität der Prothese sind unterschiedlichste Therapieformen indiziert.

Insbesondere ist für die Wahl der Frakturversorgung entscheidend, ob sich etwaig der Sitz der Prothese gelockert hat. Insofern die Prothese weiterhin fest im Knochen verankert ist, kann größtenteils von einer Revision der Prothese abgesehen werden.

Die Standardmethode bei der Versorgung von peri- oder interprothetischen Frakturen, die als Typ B1 gemäß der Vancouver-Klassifikation kategorisiert werden, stellt die winkelstabile Plattenosteosynthese dar. Oftmals führt aber gerade die Versorgung mittels Plattenosteosynthese zu einer Schwächung des prothetisch versorgten Knochens unter Bildung von Sollbruchstellen, in deren Bereich es häufig zu Folgebrüchen kommt. Je nach Befundslage kann dann der Einsatz einer Megaprothese als vollständiger Femurersatz unumgänglich sein.

Insofern die Knochenstruktur eine Osteosynthese nicht zulässt, stellt die intramedulläre Stabilisierung eine alternative Operationstechnik dar, bei der der Defekt mittels eines individuell gefertigten Interpositionsnagels überbrückt wird. Interpositionsnägel bieten eine hohe Stabilität und werden in unterschiedlichen Größen bereitgestellt.

In den Fällen von Frakturen des Typs Vancouver B1, bei denen trotz der Fraktur die Hüft- oder Knieprothese weiterhin fest im Knochen verankert ist, kann auf eine weitere Versorgungsmethode zurückgegriffen werden, bei der die implantierte Prothese im Körper verbleiben kann. Bei dieser Operationstechnik wird z.B. zur Überbrückung des Knochendefektes im Femur der Schaft einer implantierten Hüftprothese mit einem Femurnagel, der distal in den Markkanal eingesetzt wird, über ein manschettenartiges Zwischenmodul verbunden, wodurch der Schaft mechanisch verlängert werden kann. Diese Methodik bietet viele Vorteile. Einerseits kann die Dauer und Tiefe des chirurgischen Eingriffs deutlich gemindert, anderseits kann mit einer verbesserte Heilung und eine höhere Stabilität des prothetisch versorgten Knochens bei gleichzeitig reduzierter Komplikationsrate gerechnet werden.

Es existieren jedoch nur einige wenige modulare Endoprothesesysteme, bei denen die Implantat- bzw. Prothese-Komponenten derart aufeinander abgestimmt, dass eine bereits implantierte Prothese in Folgeoperationen stabil endoprothetisch erweitert werden kann.

Oftmals kann sowohl die Herkunft eines Protheseschaftes als auch dessen Geometrie und Beschaffenheit erst eindeutig festgestellt werden, wenn dieser operativ freigelegt wurde. Der Chirurg steht dann vor einer schnell zu treffenden Entscheidung, ob ein bereitgestelltes Endoprothesesystem mit dem vorgefundenen Schaft kompatibel wäre und, ob das nachträglich implantierte Endoprothesesystem mit dem Schaftende rotationsstabil verbunden werden kann.

Der Medizinproduktemarkt bietet unzählige Hüft- und Knieprothesen an, die sich in deren Konfiguration und Ausmaßen deutlich voneinander unterscheiden. Die wesentlichen Unterschiede finden sich insbesondere in der Länge und im Querschnittsprofil der Protheseschäfte sowie in deren Oberflächenbeschaffenheiten. Einige Prothesen weisen u.a. Riffelungen oder in Längsrichtung verlaufende Rillenprofile auf, die eine intramedulläre Erweiterung zu einer Endoprothese aufgrund einer nur unzufrieden herstellbaren Kraftschlüssigkeit zwischen den Protheseteilen erschweren.

Aus dem Stand der Technik sind endoprothetische Verbindungssysteme-Systeme bekannt, mit denen freiliegende Schaftenden nach einer periprothetischen Fraktur endoprothetisch verlängert werden können.

Die DE 10 2008 062 226 A1 offenbart eine Verlängerung eines proximalen Femur-Nagels. Die Verlängerung in Form eines retrograd in den in den Markraum des Femurs einbringbaren distalen Femur-Nagels weist am proximalen Ende eine Aufnahmeöffnung auf, die über das distale Ende des proximalen Femur-Nagels geschoben wird. Die Sicherung erfolgt über eine Verriegelungsschraube, die zusätzlich mit dem Femur verbunden ist.

Die US 8 668 692 B1 beschreibt ein periprothetisches Endoprothese-System für einen bereits implantierten Protheseschaftes. Dieses Endoprothese-System umfasst ein Verbindungsstück, das einen in Längsrichtung verlaufenden und dreiteilig ausgebildeten Kanal aufweist. Der proximale Bereich dieses Kanals ist für die Aufnahme des innenliegenden Schaftendes hergerichtet und ist zur Anpassung an dessen Schaftform konisch ausgebildet. Die Verbindung zwischen dem innenliegendem Schaftende und dem konisch geformten Kanalbereich erfolgt über Kraftschluss, mit oder ohne zusätzliche Einzementierung des Schaftendes. In den distalen Endbereich des Kanals kann zur endoprothetischen Verlängerung ein weiteres Implantat eingeschraubt werden.

Des Weiteren wird auf die Offenbarung der DE 39 09 182 C1 als einschlägigen Stand der Technik verwiesen.

US 2008/0255560 A1 offenbart ein intramedulläres Knochenimplantat, welches als expandierbares Stabilisierungsgerüst ausgebildet ist. Längliche plattenförmige Außenelemente sind mittels eines drahtförmigen Verbindungselements verschwenkbar mit einer starren Innenhülse verbunden. Nachdem das Implantat in einen Knochenkanal im Bereich einer Fraktur eingeführt wurde, wird es durch Verschwenken der plattenförmigen Elemente expandiert, um sich von innen gegen den Knochen abzustützen und die Fraktur zu stabilisieren.

Aufgabe der Erfindung ist es, ein implantierbares Ergänzungsbauteil für eine endoprothetische Verlängerung eines innenliegenden Implantats anzugeben, mit welchem zur Vermeidung einer Revision des innenliegenden Implantats der Kraftschluss zwischen dem innenliegenden Implantat und einer endoprothetischen Verlängerung derart verbessert werden kann, dass eine rotationsstabile Verbindung zwischen dem innenliegenden Implantat und der endoprothetischen Verlängerung erzielt werden kann.

Die Aufgabe wird mit einer implantierbaren Ausgleichsmanschette gemäß Anspruch 1 für eine Endoprothese gelöst, die zur Anbringung zwischen einem länglichen Implantatabschnitt eines ersten Implantats und einem den länglichen Implantatabschnitt des ersten Implantats umgreifenden zweiten Implantat hergerichtet ist. Die implantierbare Ausgleichsmanschette ist als Hülse ausgebildet, die einen Hülsenkörper und eine vom proximalen zum distalen Ende des Hülsenkörpers durchgehenden Durchführung zur Aufnahme des länglichen Implantatabschnitts des ersten Implantats aufweist.

Der Hülsenkörper ist aus separaten, platten- und/oder stabförmigen Ausgleichselementen gebildet, die ringförmig um die Durchführung angeordnet und in Längsrichtung des Hülsenkörpers ausgerichtet sind. Die Ausgleichselemente sind zueinander beabstandet eingerichtet, so dass zwischen zwei benachbart angeordneten Ausgleichselementen ist ein von proximal nach distal durchgehender Spalt verläuft.

Jeweils zwei benachbart angeordnete Ausgleichselemente sind durch mindestens einen faltbaren Draht zueinander beweglich miteinander verbunden, indem der die benachbarten Ausgleichselemente verbindende faltbare Draht den zwischen den benachbarten Ausgleichelementen eingerichteten Spalt überbrückt.

Der Begriff "Draht" umfasst definitionsgemäß sowohl metallische Drähte als auch Drähte aus faltbaren Kunststoffen oder synthetischen Fasern. Auch sind ummantelte Drähte mitinbegriffen.

Der Begriff "erstes Implantat" bezeichnet insbesondere Hüftprothesen, Knieprothesen oder Schulterprothese sowie Femur-, Tibia- und Gammanägel oder allgemein in das Knochenmark eingebrachte Knochennägel.

Der längliche Implantatabschnitt des Implantats ist dann beispielsweise der Protheseschaft einer Prothese, insbesondere der Endbereich eines Protheseschaftes, oder bei Knochennägeln deren Nagelendbereich.

Mit dem Begriff "zweites Implantat" ist ein Implantat bezeichnet, das dazu hergerichtet ist, mit dem länglichen Implantatabschnitt des ersten Implantats verbunden zu werden, indem der längliche Implantatabschnitt des ersten Implantats in eine z.B. im Wesentlichen hülsenförmige Aufnahmeeinrichtung im zweiten Implantat eingeführt und in dieser mittels Kraftschluss fixiert wird.

Durch das Anbringen der erfindungsgemäßen Ausgleichsmanschette auf dem Endabschnitt des ersten Implantats kann ein mangelnder Formschluss zwischen dem länglichen Abschnitt des ersten Implantats und dem zweitem Implantat aufgehoben werden.

Je nach Grad der baulichen Unterschiede kann durch den Einsatz einer erfindungsgemäßen Ausgleichsmanschette eine kraftschlüssige Verbindung von erstem und zweitem Implantat erzielt oder, insofern zwar Formschluss besteht, aber der Kraftschluss nicht ausreichend ist, die Sitzfestigkeit des Implantatverbundes derart gesteigert werden, dass die geforderte Rotationsstabilität erreicht wird.

Eine mangelnde Passform findet sich u.a. darin, dass der Innendurchmesser der hülsenförmigen Aufnahmeeinrichtung im zweiten Implantat deutlich größer als der Außendurchmesser des länglichen Implantatabschnitts des ersten Implantats ist, so dass die beiden Implantate wegen der Maßdifferenzen nicht formschlüssig zusammengefügt werden können. Ebenso ergeben sich Stabilitätsprobleme, wenn das zweite Implantat eine zylindrische Aufnahmeeinrichtung aufweist, der vorgefundene innenliegende Protheseschaft sich jedoch zum Schaftende hin verjüngt.

Durch das Aufbringen der erfindungsgemäßen Ausgleichsmanschette auf den länglichen Implantatabschnitts des ersten Implantats kann dann der Außendurchmesser vergrößert, d.h. der Endabschnitt durch eine Umfangsvergrößerung, die umlaufend oder lokal begrenzt sein kann, verdickt werden, so dass eine Passförmigkeit zur zylindrischen Aufnahmeeinrichtung im zweiten Implantat erzeugt werden kann.

Bei profilierten länglichen Implantatabschnitten eines ersten Implantats besteht vorrangig das Problem der geringen Kontaktflächengröße, die für einen rotationsstabilen Kraftschluss in einer hülsenförmigen Aufnahmeeinrichtung in einem zweiten Implantat genutzt werden kann. Bei einer derartigen Situation kann durch die Verwendung der erfindungsgemäßen Ausgleichsmanschette das Rillenprofil über einen definierten Abschnitt im länglichen Endbereich des ersten Implantats gefüllt werden, indem die Ausgleichselemente der erfindungsgemäßen Ausgleichsmanschette in eine Rille oder mehrere Rillen im Implantat eingreifen. Je nach Dimensionierung und Form der Ausgleichselemente (Querschnittsform, radiale Ausdehnung) bilden dann entweder deren der Innenwand der hülsenförmigen Aufnahmeeinrichtung zugewandten Außenseiten allein die Kontaktfläche für den rotationsstabilen Kraftschluss zwischen den beiden Implantaten oder die vorhandene Kontaktfläche am länglichen Endbereich des ersten Implantats wird durch das Auffüllen der Rillen vergrößert.

Die den Hülsenkörper bildenden Ausgleichselemente können in zwei verschiedenen Grundformen ausgebildet sein.

Bei der ersten Grundform sind die Ausgleichselemente plattenförmig ausgestaltet, wobei der Begriff "plattenförmig" definiert, dass das Ausgleichselement sowohl auf der einen Teilbereich der Außenseite des Hülsenkörpers bildenden Seite als auch auf der der Durchführung zugeordneten Innenseite (durchführungsseitig) eben bzw. planar ausgestaltet sind. Insbesondere weist ein plattenförmiges Ausgleichselement eine von proximal nach distal durchgehende polygone Querschnittsform auf, die bevorzugt eine rechteckige, rauten- oder trapezförmige Querschnittsform ist. In anderweitigen Ausführungsformen kann das Ausgleichselement auch dreieckige und mehreckige Querschnittsformen aufweisen.

Bei der zweiten Grundform sind die Ausgleichselemente stabförmig ausgestaltet, wobei der Begriff "stabförmig" definiert, dass das Ausgleichselement zumindest auf der der Durchführung zugeordneten Innenseite (durchführungsseitig) konvex ausgewölbt ist, wobei sich die Auswölbung vorteilhafterweise über die gesamte Länge des Ausgleichselements erstreckt. Mit einem stabförmigen Ausgleichselement sind insbesondere Stäbe bezeichnet, die eine von proximal nach distal durchgehende, insbesondere gleichförmig durchgehende, kreisförmige, ellipsoide oder halbkreisförmige Querschnittsform aufweisen.

In einer bevorzugten Ausführungsform verändert sich die Querschnittsform und/oder der Durchmesser über die gesamte Länge des Ausgleichselementes nicht, sondern erstreckt sich gleichförmig vom einen zum anderen Ende des Ausgleichselements.

Auch umfasst die erfindungsgemäße Lehre Ausgleichselemente, deren Querschnittsprofile sich über die Länge des Ausgleichselements verändern, bei denen beispielsweise das Querschnittsprofil von einem plattenförmigen Querschnittsprofil in ein stabförmiges Querschnittsprofi übergeht und umgekehrt.

Insbesondere kann der Hülsenkörper aus unterschiedlich ausgestalteten und/oder unterschiedlich langen Ausgleichselementen zusammengesetzt sein, indem die im Hülsenkörper angeordneten Ausgleichselemente in deren jeweiligen Querschnittsform, Querschnittserstreckung und/oder in deren Länge voneinander variieren.

In einer weiteren Ausgestaltungsform kann ein Ausgleichselement im distalen Randbereich des Hülsenkörpers einen größeren Durchmesser als in dessen gegenüberliegenden proximalen Randbereich aufweisen. Vorteilhafterweise nimmt der Durchmesser eines Ausgleichselementes über dessen Länge gleichmäßig zu. Die dadurch in Längsrichtung des Hülsenkörpers erfolgende Umfangsvermehrung kann über den gesamten Umfang eingerichtet sein, indem alle Ausgleichselemente des Hülsenkörpers proximal einen kleineren Durchmesser als distal aufweisen. Die Umfangsvermehrung kann sich aber auch nur über ein Teilsegment des Hülsenkörpers erstrecken, indem nur die dort angeordneten Ausgleichselemente im proximalen Bereich einen kleineren Durchmesser als distal aufweisen.

Mit dieser Ausgestaltungsform kann beispielsweise eine Passformdifferenz zwischen einem sich zur Implantatspitze hin verjüngenden ersten Implantats, beispielsweise einem sich in distaler Richtung verjüngenden Schaft einer Hüftprothese, und einer zylindrisch geformten Implantataufnahmeeinrichtung im zweiten Implantat ausgeglichen werden.

Um die einzelnen ringförmig angeordneten Ausgleichselemente zur Ausbildung des Hülsenkörpers zusammenzufügen, werden benachbart angeordnete Ausgleichselemente unter Ausbildung eines Spaltes über mindestens einen faltbaren Draht miteinander verbunden.

Der Grundaufbau der Ausgleichsmanschette ist im Aufbau einem Korsetts nachempfunden, bei dem die in Längsrichtung des Hülsenkörpers verlaufenden Ausgleichselemente über im Wesentlichen zur Längsrichtung querlaufende Drähte miteinander verbunden sind.

Die Spaltenweite zwischen zwei Ausgleichselementen kann durch Falten, Verknäueln oder Einknicken des Drahtes verringert werden, wobei im Verbund aller Ausgleichselemente die Spaltenweiten zwischen zwei Ausgleichselementen voneinander abweichende Maße besitzen können. So können zwei Ausgleichselemente dichter nebeneinander angeordnet sein als zwei andere im Verbund des Hülsenkörpers.

Durch Einrichten von Hülsenkörperabschnitten, in welchem Ausgleichselementen weiter als in anderen Bereichen des Hülsenkörpers zueinander beabstandet eingerichtet sind, können Kantenbereiche oder unprofilierte Bereiche im länglichen Implantatabschnitt umspannt werden, ohne dass in diesen Bereichen die für den Kraftschluss mit dem zweiten Implantat benötigte Kontaktfläche des ersten Implantat von einem Ausgleichselement belegt wird.

Die Drahtverbindung zwischen zwei Ausgleichselementen ist erfindungsgemäß nicht starr, sondern biegefähig ausgebildet, so dass zwar die Position eines jeden Ausgleichselementes im Hülsenkörper festgelegt, jedoch die Ausgleichselemente in Grenzen zueinander beweglich sind. Der Bewegungsgrad wird durch die Ausgestaltung der Verbindung von faltbarem Draht und Ausgleichselement definiert.

Die Ausgleichselemente können einerseits auf dem faltbaren Draht verschieblich gelagert sein. Zur Aufnahme eines Drahtes ist das Ausgleichselement dann vorzugsweise mit einer Bohrung oder Öse ausgestattet, durch welche der Draht geführt ist.

In einer bevorzugten Ausgestaltungsform sind Draht und Ausgleichselement jedoch fest miteinander verbunden. Die feste Verbindung kann z.B. durch Verschweißen oder Verkleben von Draht und Ausgleichselement hergestellt werden. Die Formflexibilität des Hülsenkörpers wird hierin durch die Spaltenweiten zwischen den Ausgleichselementen und durch die Verwendung eines hochbiegsamen Drahtes gewährleistet.

Durch die ausgeprägte Formflexibilität des Hülsenkörpers kann die Durchführung im Hülsenkörper an die Außenmaße und Außenform des länglichen Implantatabschnitts angepasst werden.

Im Falle einer Rillenprofilierung im länglichen Implantatabschnitt können die Ausgleichselemente im Hülsenkörper zudem derart zueinander ausgerichtet werden, dass ein oder mehrere Ausgleichselemente des Hülsenkörpers in eine Rille eines Rillenprofils im ersten Implantat eingreifen. Hierdurch wird die Rille in deren Längsrichtung über die Länge der Ausgleichsmanschette ausgefüllt, wodurch das Rillenprofil in Teilen oder vollständig nivelliert werden kann.

Vorteilhafterweise ist der den Spalt überbrückende Draht quer zur Längsachse des Hülsenkörpers ausgerichtet, so dass durch die Verdrahtung der Ausgleichselemente untereinander eine in sich bewegliche Gitterstruktur ausgebildet wird. Der Draht erfüllt in erste Linie die Funktion, vor Anbringung der Ausgleichsmanschette am länglichen Implantatabschnitt des ersten Implantats die Ausgleichselemente innerhalb des Hülsenkörpers im Verbund in Position zu halten.

In einer weiteren Ausgestaltungsform sind alle im Hülsenkörper angeordneten Ausgleichselemente von einem faltbaren Draht außenseitig radial umlaufend umgriffen, wodurch alle im Hülsenkörper angeordnete Ausgleichselemente auf gleicher Höhe miteinander verbunden sind. Es können weitere ringförmige Verdrahtungen der Ausgangselemente vorhanden sein.

Durch außenseitige Krafteinwirkung auf ein oder mehrere den Hülsenkörper bildendenden Ausgleichselemente kann die Ausgleichsmanschette zusammengedrückt werden, so dass der Hülsenkörper verformt und/oder die Durchführung im Hülsenkörper mindestens in Teilbereichen komprimiert wird.

Eine Verformung des Hülsenkörpers kann sich in einem Verbiegen einer oder mehrerer Ausgleichselemente in jedwede Richtung darstellen. Der Begriff "Verformung" umfasst auch die Komprimierung des Hülsenkörpers in radialer Richtung, die zu einer Einengung der Durchführung im Hülsenkörper zumindest über einen Teillängenbereich der Ausgleichsmanschette führt. Eine Verformung des Hülsenkörpers zeigt sich auch in einer Änderung der Spaltenweite zwischen mindestens zwei Ausgleichselementen.

Durch die Verformung kann die Ausgleichsmanschette an nahezu jedwede äußere Form eines länglichen Implantatabschnitts und/oder an dessen Oberflächenprofil angepasst werden.

Durch beispielsweise eine Gruppierung von Ausgleichselementen auf einem Oberflächenabschnitt, in welchem die Ausgleichselemente aufeinander und/oder engspaltig aneinander gelegt sein können, kann die radiale Ausdehnung des länglichen Implantatabschnitts im Bereich dieses Oberflächenabschnitts vergrößert werden. Somit ist durch Aufschieben der erfindungsgemäßen Ausgleichsmanschette auf den länglichen Implantatabschnitt über den Kontaktbereich zwischen Ausgleichsmanschette und Implantatabschnitt nachträglich und gezielt eine Veränderung der Querschnittsform des länglichen Implantatabschnitts des ersten Implantats erzeugbar.

Aufgrund der konstruktiven und funktionellen Merkmale der erfindungsgemäßen Ausgleichsmanschette ist es möglich, die Basisform des länglichen Implantatabschnitts derart zu modifizieren, dass im Verbund mit dem zweiten Implantat eine Steigerung der Rotationsstabilität erzielt wird, indem die zwischen ersten und zweiten Implantat bestehenden Differenzen in Form und Gestalt ausgeglichen werden.

Vorteilhafterweise ist die Verformung des Hülsenkörpers dauerhaft, d.h. nach Beenden der Krafteinwirkung verbleibt der verformte Hülsenkörper in dessen umgeformten Zustand.

Eine Verformung des Hülsenkörpers besteht in einer Ausgestaltungsform darin, dass die Spaltweite zwischen zwei benachbarten Ausgleichselementen verringert wird. Die Biegefähigkeit des Drahtes lässt eine Verringerung der Spaltenweiten zu. Hierbei biegt sich der Draht auf, der jedoch aufgrund seiner Faltfähigkeit durch mechanisches Ein- bzw. Umfalten eingeebnet werden kann, so dass dieser die Anbringung des zweiten Implantats nicht negativ beeinflusst. Durch das Einfalten des Drahtes wird auch erreicht, dass die Ausgleichsmanschette nach dem Aufschieben und Anformen auf dem länglichen Implantatabschnitt fest aufsitzt, so dass vor der Anbringung des zweiten Implantats die Gefahr eines unerwünschten Abgleitens ausgeschlossen ist.

Je nach Ausgestaltungsform können für einen verbesserten Eingriff in das Rillenprofil oder für eine stabilere Auflage auf der Oberseite des Implantatabschnitts die Ausgleichselemente starr ausgebildet sein.

Vorteilhafterweise besteht die Verdrahtung der Ausgleichselemente aus Drähten mit einem Durchmesser von 0,2 - 0,4 mm, insbesondere mit einem Durchmesser von 0,3 mm. Vorteilhafterweise sind die faltbaren Drähte aus Titan oder einem chirurgischem Stahl gebildet. Ein Ausgleichselement ist vorzugsweise aus Titan, einer Titanlegierung, einem chirurgischen Stahl oder einem biokompatiblen Kunststoff hergestellt.

Nachstehend wird die Erfindung anhand eines Ausführungsbeispiels näher erläutert. Die Figuren zeigen:
- Fig. 1:: Seiten- und Querschnittsansicht eines länglichen Implantatabschnitts eines ersten Implantats,
- Fig. 2:: Seitenansicht einer Ausführungsform einer erfindungsgemäßen Ausgleichsmanschette,
- Fig. 3:: Querschnittsansicht der Ausführungsform gemäß Fig. 2,
- Fig. 4:: Seitenansicht einer auf einen länglichen Implantatabschnitt gemäß Fig. 1 aufgeschobenen Ausgleichsmanschette gemäß Fig. 2 und
- Fig. 5:: Querschnittsansicht einer auf einen länglichen Implantatabschnitt gemäß Fig. 1 aufgeschobenen Ausgleichsmanschette gemäß Fig. 2.

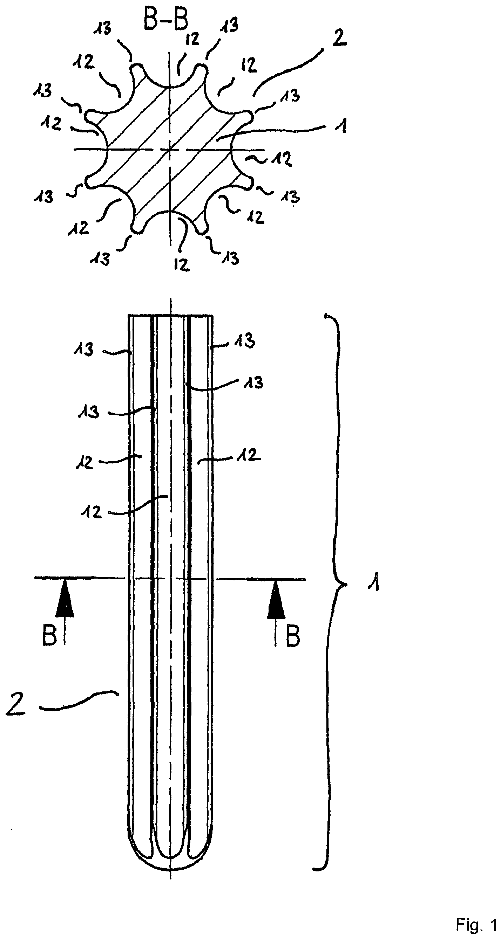

Die Figur 1 zeigt sowohl eine Seitenansicht als auch eine Querschnittsansicht B-B eines länglichen Implantatabschnitts 1 eines ersten Implantats 2. Bei dem dargestellten ersten Implantat 2 handelt es sich um einen geradlinigen Knochennagel mit einem in Längsrichtung verlaufenden Rillenprofil 12. In der Figur 1 ist der Nagelendbereich des Knochennagels abgebildet, der beispielsweise nach einer periprothetischen Fraktur operativ freigelegt wurden. Wie die Querschnittsansicht B-B zeigt, weist das wellenförmige Rillenprofil 12 des Knochennagels ausgedehnte Wellentäler mit kurzen spitzen Wellenbergen auf.

Für eine kraftschlüssige Verbindung mit einem zweiten Implantat mit einer glattwandigen Aufnahmeeinrichtung, in die das Nagelende 1 für eine endoprothetische Erweiterung eingeführt werden soll, steht für die Kraftübertragung daher nur eine sehr geringe Kontaktfläche 13 zur Verfügung, da der Kontakt nur zwischen den Wellenkämmen und der Wand der Aufnahmeeinrichtung ausgebildet werden kann.

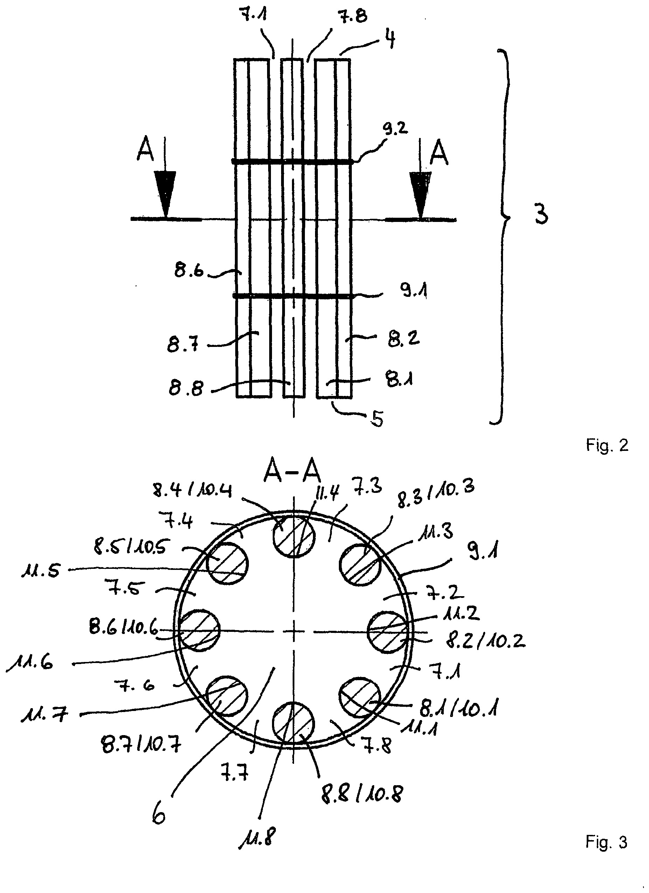

Die Figuren 2 und 3 zeigen eine Ausführungsform der erfindungsgemäßen Ausgleichsmanschette. Die Figur 3 gibt den Querschnitt A-A gemäß Fig. 2 wieder.

Die Ausgleichsmanschette ist als Hülse mit einem Hülsenkörper 3 und mit einer vom proximalen 4 zum distalen Ende 5 des Hülsenkörpers 3 durchgehenden Durchführung 6 zur Aufnahme des länglichen Implantatabschnitts 1 des ersten Implantats 2 ausgebildet.

Der Hülsenkörper 3 besteht aus mehreren ringförmig angeordneten starren Stäben als Ausgleichselemente 8.1 - 8.8, die durch zwei außenseitig den Hülsenkörper 3 umlaufende faltbare Drähte 9.1 und 9.2 miteinander verbunden sind, die mit der Außenseite eines jeden Stabes 8.1 - 8.8 fest verbunden sind. Die Stäbe 8.1 - 8.8 weisen alle die gleiche Länge und durchgehend einen kreisförmigen Querschnitt 10.1 - 10.8 auf, der über die gesamte Länge eines jeden Stabes 8.1- 8.8 konstant ist. Die Stäbe 8.1 - 8.8 sind unter Ausbildung der Spalte 7.1 - 7.8 beabstandet zueinander eingerichtet, wobei die Spaltweite der Breite eines Wellenkamms des Rillenprofils 12 entspricht. Die Drähte 9.1 und 9.2 überbrücken alle Spalten 7.1 - 7.8 und damit auch die Wellenkämme des Rillenprofils 12.

Die der Durchführung 6 des Hülsenkörpers 3 zugewandte Seite der Stäbe 8.1 - 8.8 bildet die Innenfläche 11 des Hülsenkörpers 3.

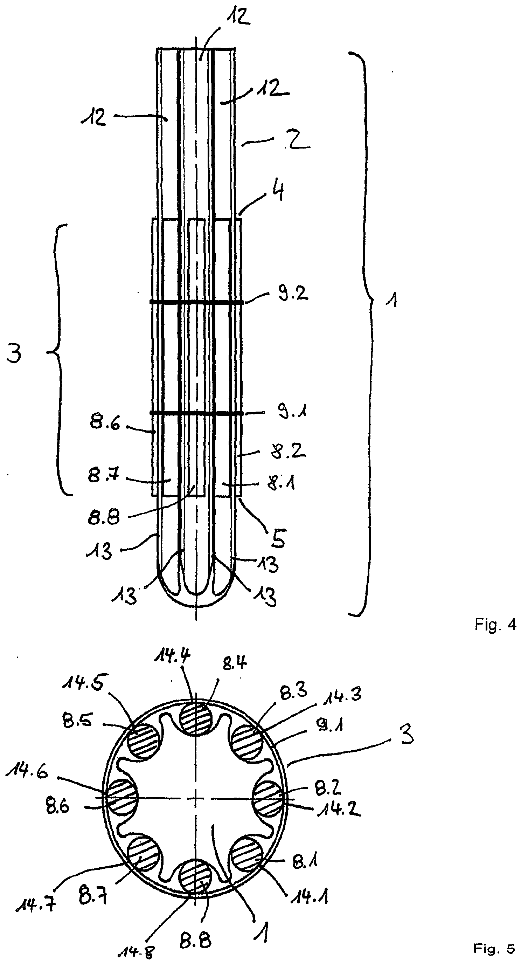

Die Figuren 4 und 5 zeigen eine auf den Nagelendbereich 2 eines Knochennagels 1 aufgeschobene Ausgleichsmanschette. Beim Aufschieben der Ausgleichsmanschette greift jeweils ein Stab 8.1 - 8.8 des Hülsenkörpers 3 in eine Rille des Rillenprofils 12 ein. Der Durchmesser der Stäbe 8.1 - 8.8 entspricht im Wesentlichen der Tiefe der Rillen des Rillenprofils 12 im länglichen Implantatabschnitt 1, so dass diese im Eingriff mit einer Rille diese im Wesentlichen ausfüllen (Fig. 5). Durch die Außenseiten 14.1 - 14.8 der Stäbe 8.1 - 8.8 wird die vorhandene Kontaktfläche 13 nahezu verdoppelt, wodurch die Rotationsstabilität zwischen dem im Bereich der Ausgleichsmanschette angesetzten zweiten Implantat gesteigert wird, da das zweite Implantat im Kraftschluss mit dem Nagelendbereich 2 nicht nur auf den Wellenkämmen des Rillenprofils 12, sondern auch auf den Außenseiten 14.1 - 14.8 der Stäbe 8.1 - 8.8 aufsitzt.
- 1: länglicher Implantatabschnitt
- 2: erstes Implantat
- 3: Hülsenkörper
- 4: proximales Ende des Hülsenkörpers
- 5: distales Ende des Hülsenkörpers
- 6: durchgehenden Durchführung im Hülsenkörper
- 7: ein von proximal nach distal durchgehender Spalt (7.1 - 7.8)
- 8: Ausgleichselemente (8.1 - 8.8)
- 9: faltbarer Draht (9.1 - 9.2)
- 10: kreisförmige Querschnittsform eines Ausgleichselements
- 11: Innenfläche des Hülsenkörpers
- 12: Rillenprofil im länglichen Implantatabschnitt des ersten Implantats
- 13: Kontaktflächen für den kraftschlüssigen Verbund mit dem zweiten Implantat
- 14: Außenseite eines Ausgleichselements (14.1 -14.8)

## Patentansprüche

1. Implantierbare Ausgleichsmanschette
zur Anbringung zwischen einem länglichen Implantatabschnitt (1) eines ersten Implantats (2) und einem den länglichen Implantatabschnitt (1) des ersten Implantats (2) umgreifenden zweiten Implantat,
wobei die Ausgleichsmanschette als eine Hülse mit einem Hülsenkörper (3) und einer vom proximalen (4) zum distalen Ende (5) des Hülsenkörpers (3) durchgehenden Durchführung (6) zur Aufnahme des länglichen Implantatabschnitts (1) des ersten Implantats (2) ausgebildet ist, **dadurch gekennzeichnet, dass**
der Hülsenkörper (3) aus separaten, platten- und/oder stabförmigen Ausgleichselementen (8.1 - 8.8) gebildet ist,
die ringförmig angeordnet und in Längsrichtung des Hülsenkörpers (3) ausgerichtet sind,
wobei zwischen zwei benachbarten Ausgleichselementen (8.1 - 8.8) ein von proximal nach distal durchgehender Spalt (7.1 - 7.8) eingerichtet ist,
und wobei benachbarte Ausgleichselemente (8.1 - 8.8) jeweils durch mindestens einen faltbaren Draht (9.1, 9.2) beweglich miteinander verbunden sind, indem der die benachbarten Ausgleichselemente (8.1 - 8.8) verbindende faltbare Draht (9.1, 9.2) den zwischen den benachbarten Ausgleichselementen (8.1 - 8.8) eingerichteten Spalt (7.1 - 7.8) überbrückt.

2. Implantierbare Ausgleichsmanschette nach Anspruch 1, **dadurch gekennzeichnet, dass**
ein stabförmiges Ausgleichselement (8.1 - 8.8) eine von proximal (4) nach distal (5) durchgehende, insbesondere gleichförmig durchgehende, kreisförmige, ellipsoide oder halbkreisförmige Querschnittsform aufweist.

3. Implantierbare Ausgleichsmanschette nach Anspruch 1, **dadurch gekennzeichnet, dass**
ein plattenförmiges Ausgleichselement (8.1 - 8.8) eine von proximal (4) nach distal (5) durchgehende, insbesondere gleichförmig durchgehende, polygone, bevorzugt eine rechteckige, rauten- oder trapezförmige Querschnittsform aufweist.

4. Implantierbare Ausgleichsmanschette nach einem der Ansprüche 1 bis 3, **dadurch gekennzeichnet, dass**
der einen Spalt (7.1 - 7.8) überbrückende faltbare Draht (9.1, 9.2) quer zur Längsachse des Hülsenkörpers (3) ausgerichtet ist.

5. Implantierbare Ausgleichsmanschette nach einem der Ansprüche 1 bis 4, **dadurch gekennzeichnet, dass**
der faltbare Draht (9.1, 9.2) die im Hülsenkörper (3) angeordneten Ausgleichselemente (8.1 - 8.8) außenseitig radial umlaufend umgreift.

6. Implantierbare Ausgleichsmanschette nach einem der Ansprüche 1 bis 5, **dadurch gekennzeichnet, dass**
der Durchmesser des faltbaren Drahtes (9.1, 9.2) 0,2 - 0,4 mm, insbesondere 0,3 mm beträgt.

7. Implantierbare Ausgleichsmanschette nach einem der Ansprüche 1 bis 6, **dadurch gekennzeichnet, dass**
die Spaltweite eines überbrückten Spaltes (7.1 - 7.8) zwischen zwei Ausgleichselementen (8.1 - 8.2) durch mechanisches Falten des faltbaren Drahtes (9.1, 9.2) mindestens im Bereich der Überbrückung verringerbar ist.

8. Implantierbare Ausgleichsmanschette nach einem der Ansprüche 1 bis 7, **dadurch gekennzeichnet, dass**
der Hülsenkörper (3) durch außenseitige Krafteinwirkung auf eine Außenseite (14.1 - 14.8) eines Ausgleichselementes (8.1 - 8.8) oder durch außenseitige Krafteinwirkung auf mehrere Außenseiten der Ausgleichselemente (8.1 - 8.8) in Richtung der Längsachse des Hülsenkörpers (3) komprimierbar und/oder verformbar, insbesondere dauerhaft komprimierbar und/oder verformbar ist.

9. Implantierbare Ausgleichsmanschette nach einem der Ansprüche 1 bis 8, **dadurch gekennzeichnet, dass**
ein Ausgleichselement (8.1 - 8.8) starr ausgebildet ist.

10. Implantierbare Ausgleichsmanschette nach einem der Ansprüche 1 bis 9, **dadurch gekennzeichnet, dass**
die im Hülsenkörper (3) angeordneten Ausgleichselemente (8.1 - 8.8) in deren jeweiligen Querschnittsform, Querschnittserstreckung, in Längsrichtung verlaufende Querschnittsverläufe und/oder in deren Länge voneinander variieren.

11. Implantierbare Ausgleichsmanschette nach einem der Ansprüche 1 bis 10, **dadurch gekennzeichnet, dass**
die Spalten (7.1 - 7.8) zwischen den Ausgleichselementen (8.1 - 8.8) voneinander abweichende Spaltenweiten aufweisen.

## Claims

1. Implantable compensation sleeve for attachment between an elongate implant portion (1) of a first implant (2) and a second implant surrounding the elongate implant portion (1) of the first implant (2),
the compensation sleeve being designed as a sheath having a sheath body (3) and a through-passage (6) that passes from the proximal end (4) to the distal end (5) of the sheath body (3) and is configured for receiving the elongate implant portion (1) of the first implant (2),
**characterized in that** the sheath body (3) is formed from separate plate-shaped and/or rod-shaped compensation elements (8.1 - 8.8) which are arranged in a ring and oriented in the longitudinal direction of the sheath body (3), a gap (7.1 - 7.8) which passes from the proximal end to the distal end being formed between two adjacent compensation elements (8.1 - 8.8), and adjacent compensation elements (8.1 - 8.8) each being movably interconnected by at least one foldable wire (9.1, 9.2), by the foldable wire (9.1, 9.2) that connects the adjacent compensation elements (8.1 - 8.8) bridging the gap (7.1 - 7.8) formed between the adjacent compensation elements (8.1 - 8.8).

2. Implantable compensation sleeve according to claim 1, **characterized in that** a rod-shaped compensation element (8.1 - 8.8) has a circular, ellipsoidal or semicircular cross-sectional shape that is continuous, in particular uniformly continuous, from the proximal end (4) to the distal end (5).

3. Implantable compensation sleeve according to claim 1, **characterized in that** a plate-shaped compensation element (8.1 - 8.8) has a polygonal, preferably rectangular, diamond-shaped or trapezoidal, cross-sectional shape that is continuous, in particular uniformly continuous, from the proximal end (4) to the distal end (5).

4. Implantable compensation sleeve according to any of claims 1 to 3, **characterized in that** the foldable wire (9.1, 9.2) bridging a gap (7.1 - 7.8) is oriented transversely to the longitudinal axis of the sheath body (3).

5. Implantable compensation sleeve according to any of claims 1 to 4, **characterized in that** the foldable wire (9.1, 9.2) surrounds the outside of the compensation elements (8.1 - 8.8) arranged in the sheath body (3), in a radially circumferential manner.

6. Implantable compensation sleeve according to any of claims 1 to 5, **characterized in that** the diameter of the foldable wire (9.1, 9.2) is 0.2 - 0.4 mm, in particular 0.3 mm.

7. Implantable compensation sleeve according to any of claims 1 to 6, **characterized in that** the gap width of a bridged gap (7.1 - 7.8) between two compensation elements (8.1 - 8.2) can be reduced at least in the region of the bridging by mechanically folding the foldable wire (9.1, 9.2).

8. Implantable compensation sleeve according to any of claims 1 to 7, **characterized in that** the sheath body (3) can be compressed and/or deformed, in particular can be permanently compressed and/or deformed, in the direction of the longitudinal axis of the sheath body (3) by external force acting on an outer face (14.1 - 14.8) of a compensation element (8.1 - 8.8) or by external force acting on a plurality of outer faces of the compensation elements (8.1 - 8.8).

9. Implantable compensation sleeve according to any of claims 1 to 8, **characterized in that** a compensation element (8.1 - 8.8) is rigid.

10. Implantable compensation sleeve according to any of claims 1 to 9, **characterized in that** the compensation elements (8.1 - 8.8) arranged in the sheath body (3) each vary in their cross-sectional shape, cross-sectional extension, cross-sectional profiles extending in the longitudinal direction and/or in their length.

11. Implantable compensation sleeve according to any of claims 1 to 10, **characterized in that** the gaps (7.1 - 7.8) between the compensation elements (8.1 - 8.8) have different gap widths.

## Revendications

1. Manchon adaptateur implantable destiné à être monté entre une partie allongée (1) d'un premier implant (2) et un second implant entourant ladite partie allongée (1) du premier implant (2),
le manchon adaptateur étant réalisé sous la forme d'un cylindre ayant un corps de cylindre (3) et un passage traversant (6) de l'extrémité proximale (4) à l'extrémité distale (5) du corps de cylindre (3) destiné à accueillir la partie allongée (1) du premier implant (2),
**caractérisé en ce que** le corps de cylindre (3) est constitué d'éléments adaptateurs (8.1 à 8.8) séparés en forme de plaque et/ou de tige, qui sont disposés en forme d'anneau et orientés dans la direction longitudinale du corps de cylindre (3),
dans lequel une fente traversante (7.1 à 7.8) du côté proximal au côté distal est disposée entre deux éléments adaptateurs (8.1 à 8.8) adjacents,
et dans lequel des éléments adaptateurs (8.1 à 8.8) adjacents sont respectivement reliés de manière déplaçable par au moins un fil pliable (9.1, 9.2), par le fait que le fil pliable (9.1, 9.2) reliant les éléments adaptateurs (8.1 à 8.8) adjacents comble la fente (7.1 à 7.8) agencée entre les éléments adaptateurs (8.1 à 8.8) adjacents.

2. Manchon adaptateur implantable selon la revendication 1, **caractérisé en ce qu'**un élément adaptateur en forme de tige (8.1 à 8.8) présente une forme en section transversale circulaire, ellipsoïdale ou semi-circulaire qui est continue, en particulier uniformément continue, du côté proximal (4) au côté distal (5).

3. Manchon adaptateur implantable selon la revendication 1, **caractérisé en ce qu'**un élément adaptateur en forme de plaque (8.1 à 8.8) présente une forme en section transversale polygonale, de préférence rectangulaire, en forme de losange ou trapézoïdale qui est continue, en particulier uniformément continue, du côté proximal (4) au côté distal (5).

4. Manchon adaptateur implantable selon l'une des revendications 1 à 3, **caractérisé en ce que** le fil pliable (9.1, 9.2) pontant une fente (7.1 à 7.8) est orienté transversalement à l'axe longitudinal du corps de cylindre (3).

5. Manchon adaptateur implantable selon l'une des revendications 1 à 4, **caractérisé en ce que** le fil pliable (9.1, 9.2) entoure radialement sur la circonférence les éléments adaptateurs (8.1 à 8.8) disposés dans le corps de cylindre (3) à l'extérieur.

6. Manchon adaptateur implantable selon l'une des revendications 1 à 5, **caractérisé en ce que** le diamètre du fil pliable (9.1, 9.2) est de 0,2 à 0,4 mm, en particulier 0,3 mm.

7. Manchon adaptateur implantable selon l'une des revendications 1 à 6, **caractérisé en ce que** la largeur d'une fente pontée (7.1 à 7.8) entre deux éléments adaptateurs (8.1 à 8.2) peut être réduite par pliage mécanique du fil pliable (9.1, 9.2) au moins dans la zone du pontage.

8. Manchon adaptateur implantable selon l'une des revendications 1 à 7, **caractérisé en ce que** le corps de cylindre (3) est compressible et/ou déformable, en particulier compressible et/ou déformable de manière permanente, dans la direction de l'axe longitudinal du corps de cylindre (3) par une force extérieure agissant sur un côté extérieur (14.1 à 14.8) d'un élément adaptateur (8.1 à 8.8) ou par une force extérieure agissant sur plusieurs côtés extérieurs des éléments adaptateurs (8.1 à 8.8).

9. Manchon adaptateur implantable selon l'une des revendications 1 à 8, **caractérisé en ce qu'**un élément adaptateur (8.1 à 8.8) est formé de manière rigide.

10. Manchon adaptateur implantable selon l'une des revendications 1 à 9, **caractérisé en ce que** les éléments adaptateurs (8.1 à 8.8) disposés dans le corps de cylindre (3) se distinguent les uns des autres par leur forme en section transversale, leur extension en section transversale, leurs parcours en section transversale s'étendant dans la direction longitudinale et/ou par leur longueur.

11. Manchon adaptateur implantable selon l'une des revendications 1 à 10, **caractérisé en ce que** les fentes (7.1 - 7.8) entre les éléments adaptateurs (8.1 à 8.8) ont des largeurs différentes.
